# EUROPÄISCHE PATENTSCHRIFT

(11) **EP 1 516 584 B1**
(45) Veröffentlichungstag und Bekanntmachung des Hinweises auf die Patenterteilung: **07.01.2009**
(21) Anmeldenummer: 04021342.3
(22) Anmeldetag: 08.09.2004
(51) Int. Cl.: A61B 5/12, H04R 25/00

(54) **Vorrichtung und Verfahren zur Bestimmung eines Hörbereichs**
Apparatus and method for determination of an audibility range
Dispositif et méthode pour détermination d'une gamme d'audibilité

(30) Priorität: 17.09.2003 DE 10343007
(43) Veröffentlichungstag der Anmeldung: 23.03.2005
(73) Patentinhaber: Siemens Audiologische Technik GmbH, 91058 Erlangen (DE)
(72) Erfinder: Chalupper, Josef, 85307 Paunzhausen (DE); Hies, Thomas, Dr., 91056 Erlangen (DE); Kortekaas, Reinier, Dr., 91052 Erlangen (DE)
(74) Vertreter: Maier, Daniel Oliver

(56) Entgegenhaltungen:
- EP-A- 1 301 060
- WO-A-01/06916
- CH-A- 678 692
- DE-A- 10 064 210
- US-A- 4 809 708
- US-A- 6 160 893

## Beschreibung

Die vorliegende Erfindung betrifft eine Vorrichtung zur Bestimmung eines Hörbereichs und ein entsprechendes Verfahren hierzu.

Bei der sogenannten Insitu-Audiometrie wird der Hörstatus des individuellen Schwerhörenden mittels Sinustöne oder schmalbandiger Rauschsignale, die im Hörgerät generiert werden, gemessen. Bei dieser Messung des Hörstatus wird eine Schwellenmessung oder eine Lautheitsskalierung durchgeführt. Bei der Schwellenmessung wird die Hörschwelle und/oder die Unbehaglichkeitsschwelle festgestellt.

Der Vorteil der Insitu-Audiometrie besteht darin, dass alle Faktoren, die einen Einfluss auf die Akustik im Gehörgang haben, im Prinzip mit berücksichtigt werden. Einen besonders starken Einfluss haben das Ohrpassstück bei einem Hinter-dem-Ohr-Hörgerät, die Hörgeräteschale bei einem In-dem-Ohr-Hörgerät und das Restvolumen zwischen dem Ohrpassstück bzw. dem Hörgerät und dem Trommelfell. Letztendlich wird bei dieser Insitu-Audiometrie mit ein und demselben Gerät gemessen und versorgt. Dadurch erhofft man sich eine bessere Schätzung des Schalldruckpegels am Trommelfell und dementsprechend eine genauere Anpassung der Verstärkung des Hörgeräts an den individuellen Hörverlust.

Derartige Verfahren zur Insitu-Audiometrie werden seit einigen Jahren von verschiedenen Hörgeräteherstellern, wie beispielsweise Widex, Phonak, Starkey, um nur einige zu nennen, in der Anpasssoftware angeboten. In Kombination mit einem tragbaren Rechner bietet sich somit eine völlig ambulante Anpassung, inklusive Audiometrie, an. Ein Anwendungsfall wäre beispielsweise die Anpassung in einem Altenheim.

Problem bei der Bestimmung des Hörstatus ist jedoch, dass laute Umgebungsgeräusche bei einer Hörschwellenmessung die leisen, vom Hörgerät generierten Töne verdecken können. Dies kann vor allem bei einer ambulanten Anpassung der Fall sein, wenn typischerweise ohne schallisolierte Hörkabine gearbeitet wird. In einer lauten Umgebung kann der Patient dann erst entsprechend laute, d. h. höher verstärkte Töne wahrnehmen, die nicht mehr verdeckt werden. Dadurch könnte der Hörverlust größer eingestuft werden als er tatsächlich ist. Dementsprechend würde die Verstärkung des Hörgeräts größer als benötigt bzw. erwünscht eingestellt werden. Hinzu kommt das Problem, dass in natürlicher Umgebung der Pegel des akustischen Umfelds stark zwischen verschiedenen Räumen und in der Regel auch über die Zeit innerhalb eines Raumes stark variiert. Dadurch ist es nicht möglich, den Verdeckungsgrad der Umgebungsgeräusche vorab zu bestimmen.

In diesem Zusammenhang ist jedoch von den Geräten der Firma Starkey bekannt, dass das vom Hörgerät selbst erzeugte Eigenrauschen in verschiedenen Verfahren berücksichtigt wird. Dieses Eigenrauschen wird unter anderem in der Endstufe durch Quantisierungseffekte hervorgerufen. In einem schallisolierten Raum bestimmt dieses Eigenrauschen die Untergrenze der theoretisch messbaren Hörschwelle. Diese Untergrenze kann in der Softwareoberfläche der Anpasssoftware angezeigt werden.

Aus der Druckschrift CH 678692 A5 ist ein Verfahren zum Messen der individuellen akustischen Verhältnisse an einem menschlichen Ohr bekannt. Dazu werden in eine so genannte Ohrmulde an der dem Trommelfell zugewandten Seite ein Hörer und ein Messmikrofon angeordnet. Die Ohrmulde wird in den Ohrkanal eingesetzt und es wird der Schalldruck unmittelbar vor dem Trommelfell gemessen.

Weiterhin zeigt das Dokument US 6,160,893 A eine spezielle Möglichkeit zur aktiven Störgeräuschunterdrückung (ANR). Dabei hat der Nutzer die Möglichkeit, zwischen mehreren voreingestellten Feedback-Controllern hin- und herzuschalten. Der Nutzer wird den für die jeweilige Situation nützlichsten Controller wählen. Zur Bestimmung der tatsächlichen minimalen Hörschwelle wird also versucht, die Auswirkungen des Störgeräuschs so weit wie möglich zu reduzieren. Wenn die Auswirkungen minimal sind, lässt sich die minimale Hörschwelle durch einen Testton ermitteln.

Darüber hinaus beschreibt die Druckschrift EP 1 301 060 A1 ein Verfahren und eine Vorrichtung zur Erfassung der Impedanz des Gehörgangs durch Messung des Schalldrucks im Gehörgang durch zwei Mikrofone. Die Messungen werden nicht mittels einer Schallsonde, sondern direkt mit der Hörhilfe durchgeführt.

Die Druckschrift US 4,809,708 offenbart ferner ein Verfahren und eine Vorrichtung zur Bestimmung der Entfernung der Spitze eines Messschlauchs vom Trommelfell. Damit werden Messungen des Schalldrucks vor dem Trommelfell aussagekräftiger. Hierbei werden Schalldruckmessungen bei zwei Positionen des Messschlauchs durchgeführt und der Schalldruck vor dem Trommelfell berechnet.

Die Aufgabe der vorliegenden Erfindung besteht somit darin, eine verbesserte Anpassung von Hörgeräten auch in nicht schallisolierten Räumen zu gewährleisten.

Erfindungsgemäß wird diese Aufgabe gelöst durch eine Vorrichtung zur Bestimmung eines Hörbereichs mit einer Messeinrichtung zum Messen eines Schalldruckpegels eines Umgebungsgeräusches, einer Schätzeinrichtung zum Schätzen eines Schalldruckpegels vor dem Trommelfell eines Patienten, der ein Hörgerät trägt, zumindest anhand der Bauform des Hörgeräts oder Ohrpassstücks und des gemessenen Schalldruckpegels des Umgebungsgeräusches sowie einer Auswerteeinrichtung zum Bestimmen eines messbaren Hörbereichs auf der Grundlage des geschätzten Schalldruckpegels vor dem Trommelfell. Dabei bedeutet der messbare Hörbereich denjenigen objektiven Bereich, in dem eine subjektive Hörschwelle messbar ist. D. h. die Untergrenze des messbaren Hörbereichs stellt die niedrigste, messbare Hörschwelle dar.

Darüber hinaus ist erfindungsgemäß vorgesehen ein Verfahren zur Bestimmung eines Hörbereichs durch Messen eines Schalldruckpegels eines Umgebungsgeräusches, Schätzen eines Schalldruckpegels vor dem Trommelfell eines Patienten, der ein Hörgerät trägt, zumindest anhand der Bauform des Hörgeräts oder Ohrpassstücks und des gemessenen Schalldruckpegels des Umgebungsgeräusches sowie Bestimmen eines messbaren Hörbereichs auf der Grundlage des geschätzten Schalldruckpegels vor dem Trommelfell.

Ein Vorteil der erfindungsgemäßen Lösung ist, dass kein zusätzliches Gerät, wie beispielsweise ein Schallpegelmesser, benötigt wird, um festzustellen, ob ein gewisser Raum sich für audiometrische Messungen eignet. Darüber hinaus kann die Umrechnung von Freifeld-Schalldruckpegel in entsprechende Mithörschwellen vom Rechner übernommen werden. Die Untergrenze des messbaren Hörbereichs kann ferner kontinuierlich während der Messung überwacht werden.

Bei einer bevorzugten Ausführungsform ist die Messeinrichtung in das Hörgerät integriert. Damit kann der Schalldruckpegel unmittelbar vor dem Hörgerät in geeigneter Weise ohne zusätzlichen Aufwand erfasst werden.

Die Messung des Schalldruckpegels der Umgebungsgeräusche kann breitbandig oder frequenzspezifisch durchgeführt werden. Die frequenzspezifische Messung hat den Vorteil, dass unterschiedliche Verdeckungen differenziert berücksichtigt werden können.

Die Schätzeinrichtung und die Auswerteeinrichtung können in eine Anpassvorrichtung zum akustischen Anpassen eines Hörgeräts an einen Patienten integriert sein. Diese Anpassvorrichtung besteht üblicherweise aus einem PC, der mit dem Hörgerät drahtlos oder drahtgebunden in Verbindung steht. Auf diesem PC, den typischerweise ein Akustiker bedient, ist die notwendige Anpasssoftware installiert.

In der Schätzeinrichtung kann ein Eigenrauschen des Hörgeräts bei der Schätzung des Schalldruckpegels vor dem Trommelfell mit berücksichtigt werden. Diese Berücksichtigung des Eigenrauschens verbessert die Schätzung des Schalldruckpegels vor dem Trommelfell, da dieser nicht nur durch Umgebungsgeräusche hervorgerufen wird.

Vorzugsweise wird in der Auswerteeinrichtung die niedrigst messbare Hörschwelle durch ein psychoakustisches Modell der Verdeckung bestimmt. Damit lässt sich aufgrund des Spektrums der Umgebungsgeräusche und des Eigenrauschens ein Verdeckungsspektrum und somit eine entsprechende Hörschwelle präzise abschätzen.

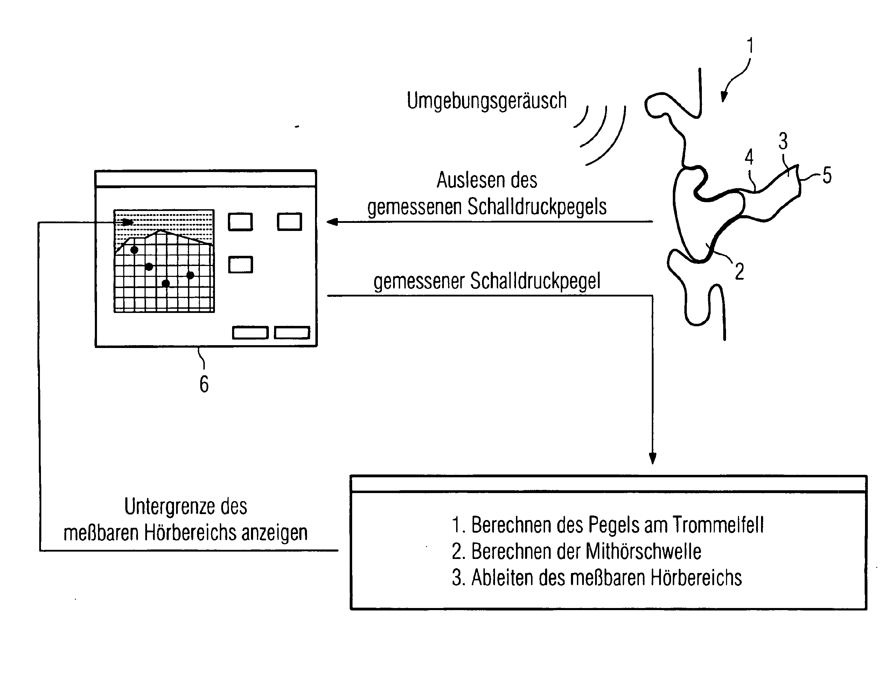

Die vorliegende Erfindung wird nun anhand der beigefügten Zeichnung näher erläutert, die eine prinzipielle Skizze der Signalverläufe zur Bestimmung eines Hörbereichs darstellt.

Das nachfolgend näher geschilderte Ausführungsbeispiel stellt eine bevorzugte Ausführungsform der vorliegenden Erfindung dar.

Ein Patient 1 trägt ein Hörgerät 2. Ein Restvolumen 3 in dem Gehörgang 4 des Patienten 1 wird einerseits durch das Trommelfell 5 und andererseits durch das Hörgerät 2 begrenzt. Dieses Restvolumen 3 bestimmt den Schalldruckpegel vor dem Trommelfell 5 maßgeblich.

Von dem Hörgerät 2 wird der Schalldruckpegel eines Umgebungsgeräuschs gemessen. Von einem PC 6, auf dem eine Anpasssoftware installiert ist, wird der gemessene Schalldruckpegel aus dem Hörgerät 2 ausgelesen. Hierzu besteht eine drahtlose oder drahtgebundene Kommunikationsverbindung zwischen dem PC 6 und dem Hörgerät 2.

Die Anpasssoftware 7 berechnet aus dem gemessenen Schalldruck den Pegel am Trommelfell 5. Daraus berechnet sie die Mithörschwelle, die sich aufgrund der durch den Schalldruckpegel am Trommelfell hervorgerufenen Verdeckung ergibt. Von der Mithörschwelle wird schließlich der tatsächlich zur Verfügung stehende Hörbereich abgeleitet. Die Untergrenze des festgestellten Hörbereichs wird auf dem PC 6 grafisch dargestellt. In dem Beispiel liegt der Hörbereich unterhalb des durchgezogenen Kurvenverlaufs. Dieser ergibt sich aus der Mithörschwelle korrigiert um die durch Umgebungsgeräusche hervorgerufene Verdeckung. Somit kann der Akustiker unabhängig von den Umgebungsgeräuschen den Hörbereich des Patienten feststellen. Anhand der Untergrenze des messbaren Hörbereichs kann der Akustiker feststellen, ob ein Raum sich für eine Bestimmung der Hörschwellen eignet. Darüber hinaus kann er feststellen, ob eine gemessene Hörschwelle eine reelle oder durch Verdeckung gegebene Schwelle ist. Damit wiederum kann er eine optimale Anpassung des Hörgeräts, insbesondere der Verstärkungen in den einzelnen Frequenzbändern, durchführen.

## Patentansprüche

1. Vorrichtung zur Bestimmung eines Hörbereichs **gekennzeichnet durch**
- eine Messeinrichtung zum Messen eines Schalldruckpegels eines Umgebungsgeräusches,
- eine Schätzeinrichtung zum Schätzen eines Schalldruckpegels vor dem Trommelfell (5) eines Patienten (1), der ein Hörgerät (2) trägt, zumindest anhand der Bauform des Hörgeräts (2) oder Ohrpassstücks und des gemessenen Schalldruckpegels des Umgebungsgeräusches sowie
- eine Auswerteeinrichtung zum Bestimmen eines messbaren Hörbereichs auf der Grundlage des geschätzten Schalldruckpegels vor dem Trommelfell (5).

2. Vorrichtung nach Anspruch 1, wobei die Messeinrichtung in das Hörgerät (2) integriert ist.

3. Vorrichtung nach Anspruch 1oder 2, wobei mit der Messeinrichtung frequenzspezifische Messungen durchführbar sind.

4. Vorrichtung nach einem der vorhergehenden Ansprüche, wobei die Schätzeinrichtung und die Auswerteeinrichtung in eine Anpassvorrichtung (6) zum akustischen Anpassen eines Hörgeräts an einen Patienten integriert sind.

5. Vorrichtung nach einem der vorhergehenden Ansprüche, wobei in der Schätzeinrichtung ein Eigenrauschen des Hörgeräts (2) bei der Schätzung des Schalldruckpegels vor dem Trommelfell mit berücksichtigbar ist.

6. Vorrichtung nach einem der vorhergehenden Ansprüche, wobei in der Auswerteeinrichtung der messbare Hörbereich durch ein psychoakustisches Modell der Verdeckung bestimmbar ist.

7. Verfahren zur Bestimmung eines Hörbereichs **gekennzeichnet durch**
- Messen eines Schalldruckpegels eines Umgebungsgeräusches,
- Schätzen eines Schalldruckpegels vor dem Trommelfell (5) eines Patienten (1), der ein Hörgerät (2) trägt, zumindest anhand der Bauform des Hörgeräts (2) oder Ohrpassstücks und des gemessenen Schalldruckpegels des Umgebungsgeräusches sowie
- Bestimmen eines messbaren Hörbereichs auf der Grundlage des geschätzten Schalldruckpegels vor dem Trommelfell.

8. Verfahren nach Anspruch 7, wobei der Schalldruck des Umgebungsgeräusches in dem Hörgerät (2) gemessen und von dort drahtlos an eine Anpassvorrichtung (6) übertragen wird, mit der das Schätzen des Schalldruckpegels vor dem Trommelfell und das Bestimmen einer Hörschwelle durchgeführt wird.

9. Verfahren nach Anspruch 7 oder 8, wobei das Messen des Schalldruckpegels frequenzspezifisch erfolgt.

10. Verfahren nach einem der Ansprüche 7 bis 9, wobei ein Eigenrauschen des Hörgeräts (2) bei der Schätzung des Schalldruckpegels vor dem Trommelfell (5) mit berücksichtigt wird.

11. Verfahren nach einem der Ansprüche 7 bis 10, wobei der messbare Hörbereich durch ein psychoakustisches Modell der Verdeckung bestimmt wird.

## Claims

1. Device for determining a hearing range
**characterised by**
- a measuring device for measuring a sound pressure level of ambient noise,
- an estimator for estimating the sound pressure level in front of the eardrum (5) of a patient (1) wearing a hearing aid (2), at least as a function of the design of the hearing aid (2) or earpiece and the measured sound pressure level of the ambient noise and
- an evaluator for determining a measurable hearing range based on the estimated sound pressure level in front of the eardrum (5).

2. Device according to claim 1, whereby the measuring device is integrated into the hearing aid (2).

3. Device according to claim 1 or 2, whereby frequency-specific measurements can be taken with the measuring device.

4. Device according to one of the preceding claims, whereby the estimator and the evaluator are integrated into an adjustment device (6) for the acoustic adjustment of a hearing aid to a patient.

5. Device according to one of the preceding claims, whereby the inherent noise of the hearing aid (2) can also be taken into account in the estimator when estimating the sound pressure level in front of the eardrum.

6. Device according to one of the preceding claims, whereby the measurable hearing range can be determined in the evaluator by means of a psycho-acoustic model of the masking.

7. Method for determining a hearing range
**characterised by**
- measurement of a sound pressure level of ambient noise,
- estimation of a sound pressure level in front of the eardrum (5) of a patient (1) wearing a hearing aid (2), at least as a function of the design of the hearing aid (2) or earpiece and the measured sound pressure level of the ambient noise and
- determination of a measurable hearing range based on the estimated sound pressure level in front of the eardrum.

8. Method according to claim 7, whereby the sound pressure of the ambient noise is measured in the hearing aid (2) and is transmitted from there wirelessly to an adjustment device (6), which is used to estimate the sound pressure level in front of the eardrum and to determine a hearing threshold.

9. Method according to claim 7 or 8, whereby the sound pressure level is measured in a frequency-specific manner.

10. Method according to one of claims 7 to 9, whereby the inherent noise of the hearing aid (2) is also taken into account when estimating the sound pressure level in front of the eardrum (5).

11. Method according to one of claims 7 to 10, whereby the measurable hearing range is determined by means of a psycho-acoustic model of the masking.

## Revendications

1. Dispositif pour déterminer une gamme d'audibilité, **caractérisé par**
- un dispositif de mesure pour mesurer un niveau de pression acoustique d'un bruit d'environnement,
- un dispositif d'estimation pour estimer un niveau de pression acoustique avant le tympan (5) d'un patient (1), qui porte un appareil de correction auditive (2), au moins à l'aide de la forme de construction de l'appareil de correction auditive (2) ou de la pièce d'adaptation à l'oreille et du niveau de pression acoustique mesuré du bruit d'environnement ainsi que
- un dispositif d'évaluation pour déterminer une gamme d'audibilité mesurable sur la base du niveau de pression acoustique estimé avant le tympan (5).

2. Dispositif selon la revendication 1, le dispositif de mesure étant intégré dans l'appareil de correction auditive (2).

3. Dispositif selon la revendication 1 ou 2, des mesures spécifiques à la fréquence pouvant être effectuées avec le dispositif de mesure.

4. Dispositif selon l'une quelconque des revendications précédentes, le dispositif d'estimation et le dispositif d'évaluation étant intégrés dans un dispositif d'adaptation (6) pour l'adaptation acoustique d'un appareil de correction auditive à un patient.

5. Dispositif selon l'une quelconque des revendications précédentes, un bruit propre de l'appareil de correction auditive (2) pouvant être pris en compte également dans le dispositif d'estimation lors de l'estimation du niveau de pression acoustique avant le tympan.

6. Dispositif selon l'une quelconque des revendications précédentes, la gamme d'audibilité mesurable pouvant être déterminée dans le dispositif d'évaluation par un modèle psychoacoustique de cache.

7. Procédé pour déterminer une gamme d'audibilité,
**caractérisé par**
- la mesure d'un niveau de pression acoustique d'un bruit d'environnement,
- l'estimation d'un niveau de pression acoustique avant le tympan (5) d'un patient (1), qui porte un appareil de correction auditive (2), au moins à l'aide de la forme de construction de l'appareil de correction auditive (2) ou de la pièce d'adaptation à l'oreille et du niveau de pression acoustique mesuré du bruit d'environnement ainsi que
- la détermination d'une gamme d'audibilité mesurable sur la base du niveau de pression acoustique estimé avant le tympan.

8. Procédé selon la revendication 7, la pression acoustique du bruit d'environnement étant mesurée dans l'appareil de correction auditive (2) et étant transmise de là sans fil à un dispositif d'adaptation (6), avec lequel l'estimation du niveau de pression acoustique avant le tympan et la détermination d'un seuil d'audibilité sont effectuées.

9. Procédé selon la revendication 7 ou 8, la mesure du niveau de pression acoustique s'effectuant de façon spécifique à la fréquence.

10. Procédé selon l'une quelconque des revendications 7 à 9, un bruit propre de l'appareil de correction auditive (2) étant pris en compte également lors de l'estimation de niveau de pression acoustique avant le tympan (5).

11. Procédé selon l'une quelconque des revendications 7 à 10, la gamme d'audibilité mesurable étant déterminée par un modèle psychoacoustique du cache.
